# EUROPEAN PATENT SPECIFICATION

(11) **EP 2 234 612 B1**
(45) Date of publication and mention of the grant of the patent: **27.06.2018**
(21) Application number: 09707091.6
(22) Date of filing: 30.01.2009
(51) Int. Cl.: A61K 31/198, A61K 31/702, A61K 31/732, A61P 31/12, A61P 31/18, A61P 35/00, A61P 37/04

(54) **COMPOSITION FOR STIMULATING NATURAL KILLER CELL ACTIVITY**
MEDIKAMENT ZUR STIMULIERUNG DER NATÜRLICHEN KILLERZELLENAKTIVITÄT
COMPOSITION STIMULANT L'ACTIVITÉ DES CELLULES TUEUSES NATURELLES

(30) Priority: 01.02.2008 WO PCT/NL2008/050060
(43) Date of publication of application: 06.10.2010
(73) Proprietor: N.V. Nutricia, 2712 HM Zoetermeer (NL)
(72) Inventor: VRIESEMA, Adrianus Johannes Maria, 3994 LS Houten (NL); VAN HELVOORT, Adrianus Lambertus Bertholdus, 6703 ED Wageningen (NL); VOS, Arjan Paul, 6721 RP Bennekom (NL); POTAPPEL - VAN 'T LAND, Belinda, 3775 KS Kootwijk (NL)
(74) Representative: Nederlandsch Octrooibureau
(86) International application number: PCT/NL2009/050045
(87) International publication number: WO 2009/096789

(56) References cited:
- EP-A- 1 321 527
- WO-A-02/47612
- WO-A-2005/067955
- US-A1- 2007 036 839
- US-B1- 6 794 495
- WATZL B ET AL: "Inulin, oligofructose and immunomodulation" BRITISH JOURNAL OF NUTRITION, vol. 93, no. 1, 2005, pages S49-S55, XP002474088
- FERRANDEZ M D ET AL: "Effects in vitro of several antioxidants on the natural killer function of aging mice - differing roles for IFN-gamma nd IL-2" EXPERIMENTAL GERONTOLOGY, vol. 34, no. 5, August 1999 (1999-08), pages 675-685, XP002474079
- DATABASE MEDLINE [Online] US NATIONAL LIBRARY OF MEDICINE (NLM), BETHESDA, MD, US; December 1994 (1994-12), MALORNI W ET AL: "Thiol supplier N-acetylcysteine enhances conjugate formation between natural killer cells and K562 or U937 targets but increases the lytic function only against the latter." XP002474080 Database accession no. NLM7721335 & IMMUNOLOGY LETTERS DEC 1994, vol. 43, no. 3, December 1994 (1994-12), pages 209-214, ISSN: 0165-2478

## Description

### FIELD OF THE INVENTION

The present invention relates to a nutritional composition for use in stimulating natural killer cell activity (NK-cell).

### BACKGROUND OF THE INVENTION

Decreased NK-cell activity is an important symptom of disease progression in oncology patients, HIV infected patients and elderly and surgical patients.

Natural killer (NK) cells are often defined by their ability to lyse certain tumour cells and virally infected cells in a nonspecific and non-histocompatibility-restricted manner. NK cells also have limited activity against bacteria, fungi, and parasites, as well as a role in regulating haematopoiesis. Human NK cells are classified morphologically as large granular lymphocytes. Phenotypically they express a variety of specific membrane-associated molecules and receptors, of which CD56 (NCAM) and CD16 are most commonly used for identification and enumeration. NK cells are believed to provide a substantial defence against viral infections and tumour cells.

It has been described in the literature that decreased NK cell activity is symptomatic in human immunodeficiency virus (HIV) infected persons, in oncology patients and in elderly or patients under stress e.g. surgery patients. The decreased NK cell activity found in HIV patients, oncology patients and elderly is supposed to have a significant effect on the effectiveness of the innate immune response in these individuals, resulting in a decreased capability to clear virus infected cells or cancer cells. In elderly this may result in increased number of viral infections and carcinoma.

Galit Alter et al; Blood 2005; 106: 3366-3369 describe the deregulation of NK cells in HIV patients.

Functional studies have measured NK cell activity against the K562 tumor cell line and demonstrate impaired NK cell cytotoxicity in elderly donors. Further evidence is derived from studies on centenarians, regarded as a model example of healthy ageing, who have been reported to have well preserved NK cell cytotoxicity, see Facchini et al. Clinical Experimental Immunology 1987, 68(2):340-347; Sansoni et al. Blood 1993, 82:2767-2773. EP 0596717 discloses the use of a nutritional supplement comprising multiple vitamins and minerals for the improvement of natural killer cell activity in elderly.

EP 0941088 discloses a method of enhancing the activity of natural killer lymphocytes and a method of increasing the basal level of natural killer activity in an animal by administering conjugated linoleic acid.

WO 2007/016132 discloses the use of two lactobacteria for the preparation of immunomodulating compositions capable of stimulating natural killer cells, in particular for the treatment and/or prevention of allergies and immunodeficiencies. WO 02/076471 discloses that the use of FOS in elderly can stimulate bifidobacteria in the faeces. No effect was shown on natural killer cells. This disclosure would lead the skilled person away from using dietary fibres for the stimulation of NK cells.

US 6,794,495 relates to a compound and method for enhancing the immune system. The compound is extensin or a combination of (a) pectin or polysaccharides found in the pectic molecule and (b) extensin.

The studies in Watzl et al. "Inulin, oligofructose and immunomodulation" vol 93(1) S49 - S55 (2005) suggest that inulin and oligofructose primarily modulate immune parameters in the gut-associated lymphoid tissue, but splenocytes are also activated by inulin and oligofructose.

### SUMMARY OF THE INVENTION

The inventors surprisingly found that natural killer cell activity can be stimulated using a nutritional composition comprising a specific dietary fibre mixture as defined in the appended claims. In particular the oral supplementation of a mixture of galactooligosaccharides, fructooligosaccharides and pectin degradation products have been shown to specifically enhance the natural killer cell activity, see examples 1, 2. As already mentioned HIV patients often have a decreased natural killer cell activity. Herein it is surprisingly shown that a nutritional composition comprising a specific dietary fibre mixture as defined in the appended claims can stimulate significantly natural killer cell activity in HIV patients. The present composition is therefore especially suitable for the treatment of decreased natural killer cell activity in HIV patients.

Oncology patients and elderly have demonstrated lower natural killer cell activity than healthy young people. This might be the cause of several immune related diseases in these patients. Therefore the nutritional composition comprising a specific dietary fibre mixture according to the invention can also be used for the treatment of decreased natural killer cell activity in oncology patients and elderly.

### DETAILED DESCRIPTION OF THE INVENTION

Decreased natural killer (NK) cell activity is a symptom of disease progression in HIV, oncology and elderly patients. The object of the invention is to increase the NK cell activity in these groups in order to prevent disease progression.

Since NK cells are capable of destroying virus infected cells and cancer cells in the human body, it is expected that these cells play a vital role in the prevention of disease progression after a viral infection has taken place. Similarly in oncology patients a strong link exists between disease progression and NK cell activity. In this patient group a decreased NK cell activity would lead to a faster growth of cancer cells. Therefore a composition that can stimulate NK cell activity in oncology patients would be very beneficial.

In HIV patients the viral load measured in the blood is a main parameter that is continuously measured during treatment. Without being bound by theory, the inventors believe that the increase in viral load is correlated with a decrease in NK cell activity. An object of the invention is therefore to provide a HIV patient with a composition that can stimulate NK cell activity for decreasing the viral load in a HIV patient.

The present invention thus relates to a composition for use in stimulating natural killer cell activity in patients infected with human immunodeficiency virus (HIV), oncology patients, said composition comprising galactooligosaccharide, fructooligosaccharide and pectin hydrolysate.

In one embodiment the present invention is for use in the treatment of patients infected with human immunodeficiency virus (HIV) or oncology patients, and said treatment comprises stimulating natural killer cell activity or increasing stimulating natural killer cell activity.

A "patient infected with human immunodeficiency virus" is a person wherein according to commonly known criteria infection with HIV has been determined. Although such a subject may not show any disease symptoms or apparently may not be ill, such a subject may also be identified as a HIV-infected patient or HIV patient. "Oncology patients" are persons who have been diagnosed to have cancer.
"Elderly" are persons of the age of 50 or more, in particular of the age of 55 or more, more in particular of the age of 60 or more, more in particular of the age of 65 or more.

The term "stimulating" as used herein in "stimulating natural killer cell activity", refers to an increase in activity of NK cells compared to the activity of NK cells prior to ingestion of the nutritional composition comprising a specific dietary fibre mixture as defined herein.

In one embodiment "stimulating natural killer cell activity" refers to an increase of natural killer cell activity. In the context of this invention an NK cell activity is determined by the commercially available test kit NKTEST®, manufactured by ORPEGEN Pharma. With the NKTEST® the cytotoxic activity of natural killer (NK) cells is quantified. In the context of this invention, NK cell activity is stimulated or increased if the activity is increased by at least 30%, preferably if the activity is increased by at least 40%, more preferably if the activity is increased by at least 50%, compared to the activity of NK cells prior to ingestion of the nutritional composition comprising a specific dietary fibre mixture as defined herein.

In one aspect present invention relates to a composition comprising galactooligosaccharide, fructooligosaccharide and pectin hydrolysate for use in the treatment of decreased natural killer cell activity in elderly.

The term "decreased" herein refers to a lower activity of NK cells in elderly compared to the activity of NK cells of healthy, non-elderly persons, e.g. compared to the average the activity of NK cells of healthy persons in the age of 30-40 years. In one embodiment decreased refers to an activity of NK cells which is at least 10% or 20% or 30% or 40% lower compared to the activity of NK cells of healthy, non-elderly persons. As already mentioned, NK cell activity is determined by the commercially available test kit NKTEST®, manufactured by ORPEGEN Pharma.

Without being bound by theory, the inventors hypothesize that the binding of oligo- and polysaccharides to Toll-like receptors or other receptors plays a role in the stimulation or activation of NK cell activity. These receptors are known of being capable of binding saccharide structures, in particular oligosaccharides and polysaccharides. The effect might be dietary fibre specific.

### Dietary fibres

Where the term "dietary fibre" is used in the present description, this refers to galactooligosaccharide, fructooligosaccharide and pectin hydrolysate. Dietary fibres as used in this invention are typically resistant to digestion and absorption in the human small intestine with preferably a complete or partial fermentation in the large intestine.

It was surprisingly found that a mixture of GOS, FOS and pectin hydrolysate had a better NK cell activity stimulating effect than the individual saccharides. Therefore the composition according to the invention comprises a mixture of three fibres, e.g. GOS and FOS and pectin hydrolysate. Preferably the ratio of the GOS, FOS and pectin is in the range of approximately 5-9:1:2-10.

Pectin is divided into two main categories: high methoxylated pectin, which is characterized by a degree of methoxylation above 50% and low methoxylated pectin having a degree of methoxylation below 50%. As used herein, "degree of methoxylation" (also referred to as DE or "degree of esterification") is intended to mean the extent to which free carboxylic acid groups contained in the polygalacturonic acid chain have been esterified (e.g. by methylation). The present acid oligosaccharide is preferably prepared from high methoxylated pectin. Preferably the acid oligosaccharides have a degree of methylation above 20%, preferably above 50 % even more preferably above 70%.

The pectin hydrolysate is preferably administered in an amount of between 10 mg and 100 g per day, preferably between 100 mg and 50 g per day, even more between 0.5 g and 20 g per day.

Dietary fibres often have a large molecular weight. The fibres used preferably have an average degree of polymerization of at least 3 and no more than 250. This is because small oligosaccharides do not bind to Toll like receptors and are therefore expected not to be effective in our model systems. This requirement does not imply that no oligosaccharides with DP 1 or 2 may be present. Most commercially available oligosaccharides consist in part of saccharides with a DP 1 and 2, while most have a DP 3 and higher. When calculating weight percentages of the oligosaccharide fraction the weight of all oligosaccharides, including the saccharides with DP 1 and 2 are used in the calculation. The total amount of fibres used in the composition therefore also depends on the source of oligosaccharides and the amount of oligosaccharides with DP 3 or higher present in the source. The maximum DP of about 250 is related to the effect on viscosity. Large molecular weight dietary fibres with a DP higher than about 250 will significantly increase the viscosity of a liquid product resulting in a decreased palatability of the product and possible difficulties during production of a liquid product.

### Nutritional compositions

In one embodiment the method or use or composition according to the present invention concerns (administering) a nutritional composition. The nutritional composition comprises galactooligosaccharide, fructooligosaccharide and pectin hydrolysate and further comprises protein, fat, digestible carbohydrates, vitamins and minerals, said composition comprising at least 10 en% milk protein, preferably at least 15 en% milk protein, based on the total composition, and said composition comprising between 10-50 en% fat, preferably between 15 and 45 en% fat, based on the total composition.

En% is short for energy percentage and represents the relative amount that a constituent contributes to the total caloric value of the composition.

The nutritional composition according to the invention comprises the dietary fibres galactooligosaccharides (GOS) and fructooligosaccharides (FOS) and pectin hydrolysate since this composition is proven to be very effective in stimulation of NK cell activity in HIV patients, see the table in example 1.

The protein is preferably selected from milk protein sources such as but not limited to skim milk, colostrum, whey, casein all preferably from bovine, goat, sheep origin, more preferably from bovine origin since this protein source the best available for the lowest price. A preferred composition comprises a mixture of whey and casein wherein the ratio whey / casein is at least 0.1, preferably at least 0.3, more preferably 0.5 and even more preferably at least 1.

Oncology, HIV and elderly patients often suffer from a loss of appetite. A preferred composition is therefore sufficiently concentrated in order to supply sufficient nutritional components, including the NK cell activity stimulating fibres, in a small volume. This will greatly improve the compliance to the product and thus the effectiveness of the product. A liquid composition according to the invention should therefore comprise per 100 ml at least 8 g protein, 15 g digestible carbohydrate, 4 g fat and 2 g dietary fibres according to the invention. Even more preferably the energy density is at least 150 kcal per 100 ml and even more preferably at least 170 kcal per 100 ml.

A problem with such nutritionally dense compositions is the increase in viscosity after sterilisation. When measured at 20 degrees Celsius at a shear rate of 100 s⁻¹, the viscosity should be lower than 100 mPa.s, preferably lower than 60 mPa.s and even more preferably below 40 mPa.s. The low viscosity is important for the taste and mouth feel of the liquid product. If the viscosity is too high, the compliance will be lower.

A desired viscosity can be achieved in two ways. One is the use of a specific protein blend that does not increase the viscosity, e.g. hydrolysed proteins or a mixture of casein whey wherein the casein to whey ratio is at least 1.5 by weight.

The other solution is using powder products. This has the advantage that all ingredients can be easily mixed with a food preferred by the patient. E.g. before consumption it can be mixed with a milk product, a fruit juice, a yoghurt, etc. No significant increase in the viscosity is then expected. Such powder composition would be used as a supplement and preferably comprises at least 15 wt% protein, preferably at least 20 wt% protein, at least 5 wt% fat preferably at least 10 wt% fat, 10 wt% dietary fibres, preferably at least 15 wt% fibres according to the invention, all based on the weight of the total composition of the powder.

The daily dose of dietary fibres preferably is at least 5 g per day, preferably at least 10 g, preferably at least 15 g and even more preferred at least 20 g per day. In order to prevent flatulence and other inconveniences the upper daily dose limit should not exceed 75 g per day and even more preferably should not exceed than 50 g per day.

A source of digestible carbohydrate is preferably included in the nutritional composition. It preferably provides about 30% to about 70% of the energy of the nutritional composition. Any suitable (source of) carbohydrate may be used, for example sucrose, lactose, glucose, fructose, corn syrup solids, maltodextrins, and mixtures thereof.

Preferably both the liquid and powder compositions further comprise a mixture of vitamins and minerals. In particular Vitamin D and zinc are important for NK cell activity and are preferably present in the upper levels of the recommended daily dose.

### Use of the compositions

The compositions according to the invention can be used for the treatment of decreased NK cell activity in elderly. Reduced NK cell activity is one of the main symptoms of a decreased capacity to respond against cells infected with a virus or cells that have become malignant. Further uses of the compositions according to the invention are as follows.

In HIV patients the compositions can be used to decrease the viral load. In particular for HIV patients it is advantageous to include N-acetyl cysteine in the compositions. In oncology patients the composition can be used for the treatment and prevention of viral infections, to kill tumour cells and for the enhancement of chemotherapy treatment. After the chemotherapy the NK cells are needed for the elimination of the remaining cancer cells. Therefore the compositions according to the invention are preferably given during and after chemotherapy.

In elderly the compositions according to the invention can be used for the treatment of decreased natural killer cell activity and for the prevention of viral infections and cancer.

**Example 1**. Stimulation of NK cell activity in HIV patients using a fibre mixture according to the invention.

Treatment protocol of HIV patients

### Design:

The study, testing the dose-response effect of a fibre mixture on NK cell activity, was randomized, double-blind and placebo-controlled in parallel group design.
Study duration was 16 weeks from the Baseline visit for each patient, of which 12 weeks of supplementation. Visits were performed for screening, baseline, and 4, 12 and 16 weeks after baseline.

### Study objective:

To determine tolerance, safety and to establish immune-modulating effects of daily consumption for 12 weeks of an oral supplement in non-symptomatic HIV seropositive adults.

The study product NR100063 was provided as a powder in individual sachets with 16 grams per sachet. The powder consisted of galactooligosaccharides (scGOS), fructooligosaccharides (IcFOS) and pectin hydrolysate (acid oligosaccharide, AOS), maltodextrin and inert sugars and was dissolved in water or juice, or mixed with yoghurt.
Study subjects consumed a total amount of 48 grams of powder - three sachets - daily for 12 weeks. Placebo received a similar number of sachets and product volume, consisting of maltodextrin and inert sugars, also dissolved in water or juice, or mixed with yoghurt.

### Study population:

57 Adult (>18 yr), male and female HIV-1 infected adults, not on HAART were randomized into three groups:
1. Double dose (N=19)
2. Single dose (N=19)
3. Placebo (N=19)

### Assessments:

Blood was drawn at baseline, Week 4, Week 12 and Week 16, for NK cell activity and safety measurements.
Stool samples were collected at baseline and after 12 weeks.
Tolerance was assessed at each visit using recall questionnaire
Compliance was assessed at each visit by counting the number of returned study product sachets and subject assessment of use of product will be made.
NK cell activity was measured using the test kit for the quantification of the cytotoxic activity of natural killer (NK) cells (NKTEST®, ORPEGEN Pharma) according to the manufacturer's instructions. A 4 hours incubation period was used for all the samples.

### Results:

**NK cell activity**

| HIV patients receiving | Baseline | 12 weeks | significance |
|---|---|---|---|
| Control | 1.4 ± 0.3 | 1.8 ± 0.4 | P= ns |
| Single dose 15g | 0.7 ± 0.3 | 2.9 ± 0.4 | P= 0.002 |
| Double dose 30g | 1.2 ± 0.4 | 2.6 ± 0.5 | P=0.026 |

The results show for the first time that the NK-cell activity is significantly increased in the HIV patients receiving single dose and double dose of the fibre mixture GOS, FOS and pectin hydrolysate both within the group and when compared to control at week 12 (p < 0.005).

### Example 2. Stimulation of NK-cell activity in fast-ageing mice

In order to investigate the effect of fibres on the NK-cell activity in aged mice, the NK-cell activity in splenocytes was measured in 8 months old fast ageing SAMP8 mice that received various doses of scGOS, IcFOS and acid oligosaccharide from pectin hydrolysate. The animals were aged on a standard rodent chow, and received the supplemented diets or control diet for the last 43 days (treatment protocol').

NK cell activity was measured by a modified "JAM" assay. The method as described by P. Matzinger in The JAM test. A simple assay for DNA fragmentation and cell death. J Immunol Methods. 1991 Dec 15;145(1-2):185-92 was used, with the modifications and specifications described here. Yac-1 cells were used as target cells, were labeled for 4-6 hours with 2.5 microCurie/ml tritiated thymidine (Perkin Elmer, Groningen, the Netherlands) and seeded at 5000 cells/well in round-bottom 96-well plates in cell culture medium (RPMI-1640 with 10% fetal calf serum). Murine splenocytes were added to the 96-well plates as effector cells in three different effector:target (E:T) ratios: 100:1, 50:1 and 25:1. Effector and target cells were incubated for 4 hours at 37 degrees Celsius in an atmosphere containing 5% carbon dioxide. After incubation, the effector and target cells were harvested on Unifilter GF/c plates (Perkin Elmer). These filterplates were dried and counted in a scintillation counter (Wallac Microbeta, Perkin Elmer) and the percentage target cell lysis was calculated as described by P. Matzinger. Statistics have been performed on the separate E:T ratios.

### Results:

At all three ratios, a significant dose-dependent increase in target cell lyses was found using linear regression analysis (slope is significantly greater than zero). The lower three plots show these regressions, statistical testing was performed in SPSS.

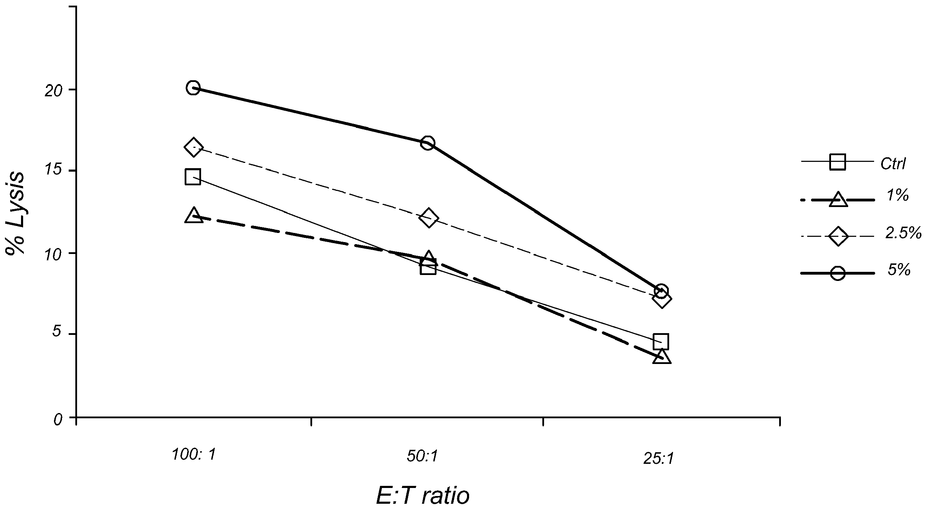

The figure shows an overview of all Effector:Target (E:T) ratios and all GFA doses. It can be seen that the NK-cell activity in the mice significantly increases with the dose of fibres indicating that the fibres can actually stimulate the NK cell activity in the aged mice.

### Reference example 3

A composition that can be used for the stimulation of NK cell activity in elderly may comprise

| | | |
|---|---|---|
| Protein | 24.9 en% | |
| | | 20%whey |
| | | 80% casein |
| Carbohydrate | 38.9 en% | |
| Fat | 36.2 en% | |
| Dietary fibre | 2.3 g/100ml | |
| Fructo oligosaccharide | | 0.8g/100ml |
| Galacto oligosaccharide | | 0.8g/100ml |
| Inulin | | |
| Vitamins | according to FSMP regulations | |
| Minerals | according to FSMP regulations | |
| Trace elements | according to FSMP regulations | |
| Energy density is between 1 and 2 kcal/ml | | |

### Example 4

A preferred composition that can be used for the stimulation of natural killer cell activity in HIV patients may comprise per 100g dry weight.

| | | |
|---|---|---|
| Dietary fibre | 5-50 g | |
| Fructo oligosaccharide | | 5% of total dietary fibre |
| Galacto oligosaccharide | | 45% of total dietary fibre |
| Pectin hydrolysate | | 50% of total dietary fibre |
| | | |
| N-acetyl cysteine | | 0.5-5g |
| Carbohydrate (not dietary fibre) | | 2-20 g |
| Fat | | 4-20 g |

### Reference example 5

A composition that can be used for the stimulation of natural killer cell activity in oncology patients may comprise per 100 ml.

| | | |
|---|---|---|
| Protein | 20-30 en% | |
| Carbohydrate | 35-45 en% | |
| Fat | 25-35 en% | |
| Fibre | 1.5 - 2.5 g | |
| Fructooligosaccharides | | 5-20 wt% of total fibre |
| Galactooligosaccharides | | 80-95 wt% of total fibre |
| Minerals | 400-1000 mg | |
| Trace elements | according to FSMP regulations | |
| Vitamins | according to FSMP regulations | |

(FSMP regulations means the most recent Food for Special Medical Purposes regulations)

## Claims

1. A composition comprising galactooligosaccharide, fructooligosaccharide and pectin hydrolysate, for use in stimulating natural killer cell activity in patients infected with human immunodeficiency virus (HIV) or oncology patients.

2. The composition for use according to claim 1, for decreasing the viral load in HIV patients.

3. The composition for use according to claim 2, wherein the composition further comprises N-acetyl cysteine.

4. The composition for use according to any one of the preceding claims, wherein the composition is a nutritional composition further comprising protein, fat, digestible carbohydrates, vitamins and minerals, said composition comprising at least 10 en% milk protein based on the total composition, and said composition comprising between 10-50 en% fat based on the total composition.

5. The composition for use according to claim 4, wherein the daily dose of galactooligosaccharide, fructooligosaccharide and pectin hydrolysate is between 5 g and 75 g.

6. A composition comprising galactooligosaccharide, fructooligosaccharide and pectin hydrolysate, for use in the treatment of decreased natural killer cell activity in elderly and for use in the prevention of viral infections and cancer in elderly.

7. The composition for use according to any of the preceding claims, wherein the ratio of the galactooligosaccharide, fructooligosaccharide and pectin hydrolysate is in the range of 5-9:1:2-10.

8. The composition for use according to claims 1-4, 6, 7, wherein the pectin hydrolysate is administered in an amount of between 10 mg and 100 g per day, preferably between 100 mg and 50 g per day, more preferably between 0.5 g and 20 g per day.

## Patentansprüche

1. Eine Zusammensetzung, umfassend Galactooligosaccharid, Fructooligosaccharid und Pektinhydrolysat, zur Verwendung der Stimulation natürlicher Killerzellaktivität bei Patienten, die mit dem Humanen Immundefizienz-Virus (HIV) infiziert sind, oder Onkologie-Patienten.

2. Die Zusammensetzung zur Verwendung nach Anspruch 1 zur Senkung der Viruslast bei HIV-Patienten.

3. Die Zusammensetzung zur Verwendung nach Anspruch 2, worin die Zusammensetzung ferner N-Acetylcystein umfasst.

4. Die Zusammensetzung zur Verwendung nach einem der vorherigen Ansprüche, worin die Zusammensetzung eine Ernährungszusammensetzung ist, die ferner Protein, Fett, verdaubare Kohlenhydrate, Vitamine und Mineralien umfasst, wobei die Zusammensetzung mindestens 10 EN% Milchprotein, bezogen auf die Gesamtzusammensetzung, umfasst und die Zusammensetzung zwischen 10 bis 50 EN% Fett, bezogen auf die Gesamtzusammensetzung, umfasst.

5. Die Zusammensetzung zur Verwendung nach Anspruch 4, worin die tägliche Dosis des Galactooligosaccharids, Fuctooligosaccharids und Pectinhydrolysats zwischen 5 g und 75 g liegt.

6. Eine Zusammensetzung, umfassend Galactooligosaccharid, Fructooligosaccharid und Pektinhydrolysate, zur Verwendung bei der Behandlung von verringerter natürlicher Killerzellaktivität bei älteren Menschen und zur Verwendung bei Vorbeugung von viralen Infektionen und Krebs bei älteren Menschen.

7. Die Zusammensetzung zur Verwendung nach einem der vorherigen Ansprüche, worin das Verhältnis von Galactooligosaccharid, Fructooligosaccharid und Pectinhydrolysat im Bereich von 5-9:1:2-10 liegt.

8. Die Zusammensetzung zur Verwendung nach einem der Ansprüche 1-4, 6, 7, worin das Pectinhydrolysate in einer Menge von zwischen 10 mg und 100 g pro Tag, vorzugsweise zwischen 100 mg und 50 g pro Tag, noch bevorzugter zwischen 0,5 g und 20 g pro Tag verabreicht wird.

## Revendications

1. Composition comprenant du galacto-oligosaccharide, du fructo-oligosaccharide et de l'hydrolysat de pectine, destinée à être utilisée pour stimuler l'activité des cellules tueuses naturelles chez des patients infectés par le virus de l'immunodéficience humaine (VIH) ou des patients en oncologie.

2. Composition à utiliser selon la revendication 1, pour diminuer la charge virale chez les patients atteint du VIH.

3. Composition à utiliser selon la revendication 2, dans laquelle la composition comprend en outre de la N-acétylcystéine.

4. Composition à utiliser selon l'une quelconque des revendications précédentes, dans laquelle la composition est une composition nutritionnelle comprenant en outre des protéines, des lipides, des hydrates de carbone digestibles, des vitamines et des minéraux, ladite composition comprenant au moins 10 en% de protéine laitière sur la base de la composition totale, et ladite composition comprenant entre 10 et 50 en% de matière grasse sur la base de la composition totale.

5. Composition à utiliser selon la revendication 4, dans laquelle la dose quotidienne de galacto-oligosaccharide, de fructo-oligosaccharide et d'hydrolysat de pectine est comprise entre 5 g et 75 g.

6. Composition comprenant du galacto-oligosaccharide, du fructo-oligosaccharide et de l'hydrolysat de pectine, destinée à être utilisée dans le traitement de l'activité réduite des cellules tueuses naturelles chez les personnes âgées et destinée à être utilisée dans la prévention d'infections virales et du cancer chez les personnes âgées.

7. Composition à utiliser selon l'une quelconque des revendications précédentes, dans laquelle le rapport du galacto-oligosaccharide, du fructo-oligosaccharide et de l'hydrolysat de pectine est compris dans la plage de 5-9:1:2-10.

8. Composition à utiliser selon les revendications 1 à 4, 6, 7, dans laquelle l'hydrolysat de pectine est administré en une quantité comprise entre 10 mg et 100 g par jour, de préférence entre 100 mg et 50 g par jour, de manière plus préférée entre 0,5 g et 20 g par jour.
